Europäisches Patentamt

(19) European Patent Office

Office européen des brevets

(11) Veröffentlichungsnummer: **0 141 953 B1**

(12) **EUROPÄISCHE PATENTSCHRIFT**

(45) Veröffentlichungstag der Patentschrift:
09.12.87

(21) Anmeldenummer: **84110565.3**

(22) Anmeldetag: **05.09.84**

(51) Int. Cl.⁴: **H 04 R 25/00,** G 09 B 21/00,
A 61 F 11/04

(54) **Verfahren und Vorrichtung zur Übertragung akustischer Informationen als fühlbare Vibrationen.**

(30) Priorität: **19.09.83 DE 3333776**

(43) Veröffentlichungstag der Anmeldung:
**22.05.85 Patentblatt 85/21**

(45) Bekanntmachung des Hinweises auf die Patenterteilung:
**09.12.87 Patentblatt 87/50**

(84) Benannte Vertragsstaaten:
**AT CH DE FR GB IT LI NL SE**

(56) Entgegenhaltungen:
**EP - A - 0 077 688**
**DE - A - 2 739 609**
**DE - A - 2 908 999**
**DE - B - 1 261 169**
**FR - A - 2 469 039**
**GB - A - 1 340 105**

**ELECTRICITE ELECTRONIQUE MODERNE, Band 44, Nr. 281, Juni/Juli 1974, Seiten 10-13, Paris, FR; C. VIGNERON et al.: "Un diviseur de fréquences audibles et son utilisation en prothèse auditive"**

(73) Patentinhaber: **Siemens Aktiengesellschaft Berlin und München, Wittelsbacherplatz 2, D-8000 München 2 (DE)**

(72) Erfinder: **Busch, Dieter, Sportplatzstrasse 9, D-8551 Forchheim (DE)**
Erfinder: **Harless, Friedrich, Kleinreuther Weg 40, D-8500 Nürnberg (DE)**

**Beschreibung**

Die Erfindung betrifft Verfahren zur Übertragung akustischer Informationen als fühlbare Vibrationen nach dem Oberbegriff des Patentanspruchs 1 und Vorrichtungen zur Durchführung dieses Verfahrens. Derartige Verfahren und Vorrichtungen sind etwa bekannt aus der US-A- 42 89 935.

Bei der vibratorischen Übertragung von akustischen Signalen, etwa Sprache, wird die Haut (z.B. am Armgelenk) mechanisch gereizt. Der Fühlbereich der Haut für solche mechano-kutane Reizungen beginnt bei ziemlich tiefen Frequenzen, etwa bei solchen in der Grössenordnung von 20 Hz. Der obere Bereich liegt aber im Vergleich zum Gesamtbereich der hörbaren Frequenzen relativ niedrig, bei etwa 1,2 kHz (vgl. z.B. Funkschau 11 (1983) Seite 53). Stimmlose Laute, wie z.B. s, sch, ch, deren akustische Erscheinung vorwiegend Frequenzen grösser als 2 kHz aufweisen, sind daher durch lineare Übertragung durch Reizung der Haut mittels Vibrationen nicht fühlbar zu machen. Bei bekannten Geräten hilft man sich durch Verwendung eines Mikrofons, welches nahe an den Mund gehalten wird, so dass der Blasstrom vom Mund auf der Mikrofonmembrane ein für die stimmlosen Laute charakteristisches Rumpelgeräusch erzeugt, welches dann fühlbar gemacht werden kann.

Bei einer tragbaren Vorrichtung zur Übertragung von akustischen Signalen als Vibrationen, d.h. bei Garäten, die als Kommunikationshilfe, d.h. eine Art Hörgerät, eingesetzt werden sollen, ist ein extrem nahes Besprechen des Mikrofons einerseits aus kosmetischen Gründen und ausserdem, weil auch stimmlose Laute der näheren und weiteren Umgebung übertragen werden sollen, nicht möglich. Bei derartigen Geräten wird das Mikrofon wie bei üblichen Taschen-Hörgeräten fest mit der Verstärkereinheit verbunden oder als an Bekleidung anbringbares Mikrofon ca. 20 bis 30 cm vom Mund entfernt getragen.

Nach der US-A 42 89 935 ist ein Gerät bekannt, bei welchem zur Erzielung guter Verständlichkeit und Vereinfachung des bei Hörgeräten für äusserst Schwerhörige sonst üblichen apparativen Aufbaus eine Aufteilung der zu übertragenden Signale in mehrere Frequenzbänder vorgenommen und zur Modulierung von Wechselspannungen (Töne) verwendet wird, die dann zusammen mit vom Mikrofon kommenden Signalen nach Verstärkung einem Hörer oder auch einem Vibrator (vibrotaktiler Reizgeber) zugeleitet werden. Für stimmlose Laute wird dabei von dem Linienspektrum auf ein Rauschspektrum bzw. ein Linienspektrum umgeschaltet. Der für eine derartige Umsetzung vorgesehene Vocoder, dh. der hohe Aufwand, ist bisher einer Einführung in die Hörgerätetechnik hinderlich gewesen.

Der Erfindung liegt die Aufgabe zugrunde, bei einem Verfahren nach dem Oberbegriff des Anspruchs 1 und Vorrichtungen zur Durchführung dieses Verfahrens eine handliche und unter den üblichen Hörgerätebedingungen betreibbare Methode anzugeben. Diese Aufgabe wird erfindungsgemäss durch das im Kennzeichen des Anspruchs 1 angegebene Verfahren und die in den Unteransprüchen angegebenen Vorrichtungen gelöst.

Die Erfindung sieht eine Transformation der charakteristischen hohen Frequenzen der Zischlaute in den Bereich fühlbarer Frequenzen vor. Dazu kann das akustische Signal in einem Mikrofon in eine elektrische Signalfolge umgewandelt und in üblicher Weise verstärkt dem Vibrator zugeführt werden, wobei das Signal im Übertragungsweg abgezweigt und daraus mittels Filter die hohen, den Zischlauten entsprechenden Frequenzen ausgesondert werden. Diese für die stimmlosen Laute charakteristischen Frequenzanteile werden dann einer Intermodulationsverstärkung zugeführt, so dass Summen- und Differenzprodukte der Frequenzanteile erhalten werden. Das Ergebnis der Intermodulationsverstärkung, d.h. die Summen- und Differenzfrequenzen der stimmlosen Laute, wird schliesslich dem Grundsignalfluss wieder zugeführt. Zur Vermeidung einer Überlastung des Endverstärkers durch die hochfrequenten Summenfrequenzen ist es in der Regel zweckmässig, die niederfrequenten Anteile, die unterhalb von 1.000 Hz liegen und über einen Vibrator fühlbar gemacht werden können, auszufiltern und nur diese weiterzuleiten. Am Ende wird jedenfalls neben den bei bekannten Geräten auftretenden fühlbaren Vibrationen eine für die stimmlosen Laute charakteristische Vibrationsserie erhalten. Dabei ist nur die Aufnahme der Zischlaute selbst erforderlich und kein Blasgeräusch am Mikrofon, so dass auch bei tragbaren Geräten eine einwandfreie Erkennung der Zischlaute möglich wird. Andererseits ist zur Durchführung des Verfahrens lediglich ein Aufbau erforderlich, der mit wenigen einfachen Bauteilen auskommt, die mit den üblichen Hörgerätebatterien betreibbar sind.

In einem Ausführungsbeispiel der Erfindung werden die akustischen Signale in einem Mikrofon aufgenommen und in einem Vorverstärker mit Limiter verstärkt. Der Limiter bietet dabei Gewähr dafür, dass am Ausgang des Vorverstärkers ein unverzerrtes Signal für die weitere Signalverarbeitung zur Verfügung steht, insbesondere, dass durch beim Begrenzer entstehende Oberwellen keine Signale entstehen, die künstlich das Vorhandensein stimmloser Laute simulieren könnten. Das so verstärkte Signal gelangt dann auf direktem Weg über einen Lautstärkeregler und Endverstärker zum Vibrator.

Zur Transformation der für die stimmlosen Laute charakteristischen hohen Frequenzanteile über 2 kHz wird nach dem Vorverstärker das Sprachsignal abgezweigt und zunächst über ein Hochpassfilter gefiltert. Ein Frequenzübergang an der Filtergrenze von 12 dB/Oktave reicht aus, weil der erwünschte Effekt mit verhältnismässig niedrigem Aufwand deutlich erzielt wird. So wird durch Ausfilterung der Frequenzen in einer Grössenordnung von 2 kHz und höher liegend ein zur weiteren Verarbeitung für die stimmlosen Laute charaktristisches Frequenzband erhalten. Die darin enthaltenen Frequenzanteile sind keine diskreten Spektrallinien, sondern ähneln eher einem hochpassgefilterten, breitbandigen Rauschsignal (z.B. hochpassgefiltertes weisses Rauschen). Das vorliegende Rauschen enthält aber trotzdem eine Frequenzverteilung, die für die jeweiligen Zischlaute charakteristisch ist.

In einer auf das Filter folgenden Verstärkerstufe wird das Signal auf einen Pegel gebracht, der ausreicht, dass in der nachfolgenden Intermodulationsstufe die Aussteuerung so erfolgt, dass über einen möglischst breiten Bereich ein lineares Signal erhalten wird. In dieser Stufe erzeugt man aus dem vorher ausgefilterten Rauschen an einer nichtlinearen Kennlinie Intermodulationsprodukte, d.h. die hohen Frequenzanteile am Eingang der Intermodulationsstufe treten an deren Ausgang zusätzlich als Summen- und Differenzfrequenzen auf. Die nichtlineare Kennlinie der Intermodulationsstufe kann dabei einer quadratischen Übertragungscharakteristik entsprechen. Dies ist vorteilhaft, weil ausschliesslich Summen- und Differenzfrequenzen der Grundwellen entstehen, aber keine Summen- und Differenzfrequenzen des Vielfachen der Grundwellen. Dies würde eine Differenzierung der stimmlosen Laute erschweren.

Von den Differenzfrequenzen fällt ein Teil in den niederfrequenten Frequenzbereich, der unterhalb von 1 kHz liegt und deshalb mittels eines Vibrators fühlbar gemacht werden kann. Diese Frequenzen werden daher, wie oben erwähnt, zweckmässigerweise in einem Tiefpassfilter, wofür ebenfalls ein Filter zweiter Ordnung ausreicht, abgetrennt. Das so erhaltene Signal kann dann noch in einer Verstärkerstufe in seinem Pegel demjenigen des ursprünglichen Signals angepasst und in einer Summationsstufe diesem ursprünglichen Signal wieder zugeführt werden, so dass über den Lautstärkeregler und die Endstufe der Vibrator ein Signalgemisch erhält, welches neben den bei einfacher Verstärkung vorliegenden Frequenzen auch die transformierten Frequenzen der Zischlaute enthält.

Als Hoch- und Tiefpassfilter sind in den abgezweigten Signalwegen etwa beliebige aktive oder passive RC-Filter zweiter Ordnung verwendbar. Die Grenzfrequenz des Hochpassfilters sollte im Bereich von ca. 2 kHz bis 4 kHz liegen. Ab 2 kHz sind im Frequenzspektrum der Sprache charakteristische Frequenzanteile der stimmlosen Laute vorhanden. Bei einer Grenzfrequenz grösser 4 kHz besteht die Gefahr, dass die verschiedenen stimmlosen Laute nicht mehr differenziert werden können. Ein Tiefpass ist an sich nicht zwingend notwendig, weil der Fühlbarkeitsbereich der Haut bei ca. 1 kHz endet. Ein solches Filter ist aber zweckmässig, um eine Übersteuerung der Endstufe mit Differenzfrequenzen ausserhalb des benötigten Frequenzbereiches zu vermeiden. Die geeignete Grenzfrequenz liegt im Bereich von ca. 200 Hz bis 1 kHz. Die Übertragung der Differenztöne im Bereich bis ca. 200 Hz enthält die wichtige Information (vergleichbar mit den Blasgeräuschen eines mundnahen Mikrofons) und muss stattfinden können. Die Übertragung von Frequenzen ab 1 kHz ist unötig, weil man sich damit ausserhalb des fühlbaren Bereiches der Haut befindet.

Zur Erzeugung des Intermodulationssignals ist eine Stufe anwendbar, die einen Operationsverstärker enthält, über den das Signal an eine Diode gelangt. Dort wird das Intermodulationssignal überwiegend erzeugt, indem eine Multiplikation der Summe der Frequenzen durch die nichtlineare Kennlinie der Diode stattfindet. Da ein gewünschter Aussteuerungsbereich, d.h. ein solcher im Arbeitsbereich der Diode, die Aussteuerung bei grossen Signalen zu überproportional grossen Ausgangssignalen führen kann, ist es zweckmässig, dieses Ansteigen der Kennlinie etwa über einander entgegengesetzt geschaltete Dioden zu kompensieren. Eine lineare Kennlinie der Intermodulationsprodukte lässt sich durch entsprechende Dimensionierung der übrigen Bauelemente erreichen.

Weitere Einzelheiten und Vorteile der Erfindung werden nachfolgend anhand der in den Figuren dargestellten Ausführungsbeispiele weiter erläutert.

In der Figur 1 ist ein Prinizipschaltbild eines mit einem Vibrator ausgerüsteten Hörgerätes dargestellt und

in der Figur 2 ein Schaltungsbeispiel für die in Figur 1 enthaltene Intermodulationsstufe.

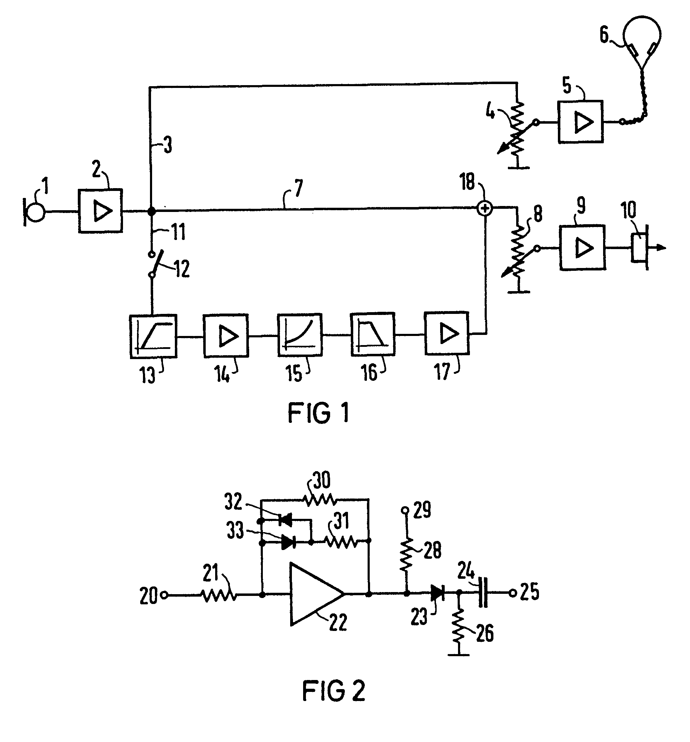

In der Figur 1 ist mit 1 ein Mikrofon bezeichnet, das an einem Limiterverstärker 2 angeschlossen ist. Von diesem führt eine Leitung 3 über einen Lautstärkeregler 4 und einen Endverstärker 5 zu einem Kopfhörer 6. Über eine weitere Leitung 7 und einen Lautstärkeregler 8 sowie einen Endverstärker 9 besteht eine weitere Verbindung des Vorverstärkers 2 zu einem Vibrator 10. Schliesslich ist noch eine Leitung 11 vorhanden, die über einen Schalter 12 zu einem Hochpassfilter 13 führt, welches nur Frequenzen durchlässt, die über 2 kHz liegen. Diese Frequenzen gelangen dann an einen Verstärker 14, vom dem aus sie zu einem Intermodulationsverstärker 15 gelangen. Die Ausgangssignale aus dem Intermodulationsverstärker 15 werden dann über ein Tiefpassfilter 16 und einen Verstärker 17 einem Summationspunkt 18 zugeführt, über welchen das mittels der Leitung 11 abgeleitete und in den Elementen 13 bis 17 bearbeitete Signal dem ursprünglichen, mittels der Leitung 7 übertragenen Signal wieder beigefügt wird.

Der zwischen dem Mikrofon 1 und dem Hörer 6 liegende Signalverlauf entspricht dem in üblichen Hörgeräten. Dabei wird das Signal im Mikrofon 1 aufgenommen, in eine elektrische Signalfolge umgewandelt und im Vorverstärker 2 verstärkt. Dieses Signal gelangt dann, nachdem es den Lautstärkeregler 4 passiert hat, in den Endverstärker 5, von dem aus dann der Kopfhörer 6 in Tätigkeit gesetzt wird. Dieser Zweig kann mit seinen Teilen 3 bis 6 wegfallen, wenn es sich um ein Gerät handelt, welches nur für die Versorgung von Personen erstellt wird, die keinerlei Hörvermögen mehr haben; dann reicht es auch, wenn das im Vorverstärker 2 behandelte Signal direkt über die Leitung 7 und einen Lautstärkeregler 8 einem Endverstärker 9 zugeführt wird, von welchem aus der Vibrator 10 betrieben wird.

Die erfindungsgemäss verbesserte Form eines Gerätes, wie es durch den Aufbau der Teile 1, 2 und 7 bis 10 gegeben ist, wird erhalten, indem zwischen dem Vorverstärker 2 und dem Lautstärkeregler 8 über eine Leitung 12 das Signal abgeleitet wird. Diese Signal kann dann über den Schalter 12 an die Bearbeitungsstufen 13 bis 17 angeschlossen werden, die dann das bearbeitete Signal dem ursprünglichen, über die Leitung 7 fliessenden Signalweg wieder zufügen.

Bei geschlossenem Schalter 12 wird im Hochpassfiler, dessen Grenzfrequenz bei 2 kHz liegt, der über der Grenzfrequenz liegende Anteil des Signals ausgefiltert und dann im Verstärker 14 auf einen Pegel gebracht, der zur Versorgung der Intermodulationsstufe 15 ausreicht. Im Filter 16 werden dann die Summationssignale der Stufe 15 ausgesondert und wegen der Grenzfrequenz von 400 Hz des Filters 16 nur die im Vibrator 10 fühlbar machbaren Anteile durchgelassen. Schliesslich werden diese Anteile im Verstärker 17 auf einen Pegel gebracht, der die Einfügung des zwischen 13 und 17 behandelten Signals in den ursprünglichen Fluss der Leitung 7 ohne Störung ermöglicht.

Als Filter 13 ist dabei ein aktives RC-Filter zweiter Ordnung eingesetzt, dessen Grenzfrequenz bei 2 kHz liegt und als Tiefpassfilter ein solches zweiter Ordnung, dessen Grenzfrequenz bei 400 Hz liegt.

Die Intermodulationsstufe 15 weist gemäss Figur 2 nach ihrem Eingang 20 einen Widerstand 21, einen Operationsverstärker 22, eine Diode 23 sowie einen Kondensator 24 auf, bevor die Verbindung am Ausgang 25 endet. Zwischen der Diode 23 und dem Kondensator 24 liegt eine Abzweigung über einen Widerstand 26 an Masse. Zwischen dem Verstärker 22 und der Diode 23 liegt über einen Widerstand 28 ein Anschluss 29 zum positiven Pol der Betriebsstromquelle. Der Operationsverstärker 22 weist eine Überbrückung über einen Widerstand 30 auf. Ausserdem ist eine weitere Überbrückung des Verstärkers 22 über eine Verbindung vorhanden, welche einen Widerstand 31 und einander entgegengerichtet gepolte Dioden 32 und 33 enthält.

Für ein Gerät gemäss Figur 1, welches mit einer Stromquelle von 4 bis 6 Volt betrieben wird, ergibt sich eine zweckmässige Ausgestaltung des Intermodulationsverstärkers 15 mit einem Operationsverstärker, der durch eine Mindestversorgungsspannung von 3 Volt, obere Grenzfrequez 20 kHz bei einer Verstärkung von 30 dB, charakterisiert ist und einer Bestückung mit einem Widerstand 21 von 10 kOhm, einem Widerstand 26 von 100 kOhm, einem Widerstand 28 von 3,3 kOhm, einem Widerstand 30 von 330 kOhm und einem Widerstand 31 von 22 kOhm. Der Kondensator 24 weist eine Kapazität von 1 µF auf. Die Dioden 23, 32, 33 sind handelsübliche Kleinsignal-Siliziumdioden mit ca. 400 mW zulässiger Leistung.

Bei der vorgenannten Dimensionierung der Bauteile der Intermodulationsstufe 15 bewirken die Widerstände 21 zusammen eine Verstärkung des Verstärkers 22 von 33 dB (= ca. 30 dB). Die Diode 23 ergibt wegen nichtlinearer, teilweise quadratischer Kennlinie das eigentliche Intermodulationssignal, welches dann durch den Kondensator 24 auf einen Belastungswiderstand ausgekoppelt wird. Um das Intermodulationssignal möglichst gross zu halten, sollte dieser Lastwiderstand in der Grössenordnung von 100 kOhm liegen. Der Widerstand 26 hält den Kondensator 24 auf festem Potential. Der Kondensator 27 und der Widerstand 35, der über den Anschluss 34 an die negative Batteriespannung gelegt wird, legen den Arbeitspunkt der Diode 23 fest. Dadurch kann die Übertragungskennlinie verändert werden (z.B. Erhöhung der niedrigen Pegel durch Widerstand 35 oder Verschiebung der Kennlinie parallel durch Kondensator 24). Durch den Anschluss der positiven Versorgungsspannung bei 29 über den Widerstand 28 wird erreicht, dass der Verstärker 22 in seinem bestimmungsgemässen Betriebsbereich arbeitet. Der Widerstand 31 in Reihe mit den einander gegenpolig geschalteten Dioden 32 und 33 ergibt, dass der überproportionale Anstieg der Übertragungskennlinie des Intermodulationsverstärkers bei höheren Pegeln kompensiert und damit die Übertragungskennlinie linearisiert wird.

## Patentansprüche

1. Verfahren zur Übertragung akustischer Informationen als fühlbare Vinbrationen, die aus den verstärkten, in eine elektrische Signalfolge umgewandelten gesamten Informationen (Grundsignal) und einem aus dem darin enthaltenen hohen Frequenzanteil abgeleiteten Signal in einem Vibrator (10) erhalten werden, dadurch gekennzeichnet, dass die Ableitung des Signals aus dem hohen Frequenzanteil durch die Bildung der Differenzfrequenzen der Signalkomponenten, aus denen sich der hohe Frequenzanteil zusammensetzt, erfolgt.

2. Verfahren nach Anspruch 1, dadurch gekennzeichnet, dass die Ableitung über eine Stufe (15) mit einer Kennlinie nichtlinearer Charakteristik, insbesondere quadratischer Charakteristik erfolgt.

3. Verfahren nach Anspruch 1, dadurch gekennzeichnet, dass die hohen Frequenzanteile dem Verstärker der Signalfolge zwischen Vorverstärker (2) und Endverstärker (9) entnommen, einer Summen- und Differenzfrequenzen bildenden Stufe (15) zugeführt und schliesslich auf einen dem Grundsignal entsprechenden Pegel gebracht und mit diesem Signal zusammen dem Endverstärker mit Lautstärkeregler (8) zugeleitet werden und dass dann das Signalgemisch in einem Vibrator (10) in fühlbare Vibrationen umgesetzt wird.

4. Vorrichtung zur Durchführung des Verfahrens nach Anspruch 3, wobei die akustischen Informationen in einem Mikrofon (1) aufgenommen werden, welches an einem Vorverstärker (2) angeschlossen ist, der über einen Endverstärker (9) an einem Vibrator (10) liegt, und dass zwischen den beiden Verstärkerteilen ein weiterer Signalkanal abzweigt, der nacheinander ein Hochpassfilter (13), einen Verstärker (14), eine Stufe (15) mit einer Kennlinie nichtlinearer Charakteristik, ein Tiefpassfilter (16), und einen weiteren Verstärker (17) enthält.

5. Vorrichtung nach Anspruch 4, dadurch gekennzeichnet, dass die Stufe (15) mit der Kennlinie nichtlinearer Charakteristik einen Operationsverstärker (22) enthält, dem eine Diode (23) nachgeschaltet ist.

6. Vorrichtung nach Anspruch 5, dadurch gekennzeichnet, dass der Operationsverstärker eine Mindestversorgungsspannung von 3 Volt und eine obere Grenzfrequenz von 20 kHz bei Verstärkung 30 dB besitzt und die Diode als Kleinsignal-Siliziumdiode mit 400 mW zulässiger Leistung dimensioniert ist, wobei der Operationsverstärker über zwei parallelliegende und entgegengesetzt zueinander gepolte Dioden in Serie zu einem Widerstand überbrückt ist.

7. Vorrichtung nach den Ansprüche 5 und 6, dadurch gekennzeichnet, dass der Operationsverstärker (22) über einen Widerstand (21) am Eingang der Stufe liegt und dass auf die Diode zum Ausgang hin ein Kondensator (24) folgt, wobei zwischen Diode und Kondensator über einen Widerstand (26) eine Verbindung zur Masse vorhanden ist, ausserdem ist zwischen dem Operationsverstärker und dem Kondensator über einen Widerstand (28) ein Anschluss zum positiven Pol der Betriebsstromquelle vorhanden.

8. Vorrichtung nach Anspruch 4, dadurch gekennzeichnet, dass das Hochpassfilter (13) eine Grenzfrequenz hat, die im Bereich 2 kHz bis 4 kHz liegt und das Tiefpassfilter (16) eine solche im Bereich von 200 Hz bis 1 kHz.

**Claims**

1. A method of transmitting acoustic items of information as perceptible vibrations produced by a vibrator (10) and obtained from complete information (basic signal), which has been amplified and converted into an electrical signal sequence, and from a signal acquired from a high frequency component contained therein, characterized in that the acquisition of the signal from the high frequency component is achieved by forming the difference frequencies of the signal components from which the high frequency component is composed.

2. A method as claimed in Claim 1, characterised in that the acquisition of the signal from the high frequency component takes place via a stage (15) having a non-linear characteristic curve, in particular a quadratic characteristic.

3. A method as claimed in Claim 1, characterised in that the amplifier high frequency components are obtained from the signal sequence between the preliminary amplifier (2) and the output amplifier (9), supplied to a stage (15) which forms sum-and-difference frequencies, and are finally brought to a level corresponding to that ot the basic signal and are supplied to the output amplifier and loudspeaker regulator (8) together with this signal, and that the signal mixture is then converted into perceptible vibrations in a vibrator (10).

4. A device for carrying out the method claimed in Claim 3, where the items of information are recorded acoustically in a microphone (1) connected to a preliminary amplifier (2) which is connected via an output amplifier (9) to a vibrator (10), and that a further signal channel that branches off between the two amplifier components contains in series a high-pass filter (13), an amplifier (14), a stage (15) having a non-linear characteristic curve, a low-pass filter (16) and a further amplifier (17).

5. A device as claimed in Claim 4, characterised in that the stage (15) which has the non-linear characteristic curve contains an operational amplifier (22) followed by a diode (23).

6. A device as claimed in Claim 5, characterised in that the operational amplifier has a minimum supply voltage of 3 volts and an upper cut-off frequency of 20 kHz at an amplification of 30 dB, and the diode is dimensioned as a low-level signal silicon-diode with 400 mW permissible power, where the operational amplifier is bridged via two parallel diodes of opposing polarity in series with a resistor.

7. A device as claimed in Claims 5 and 6, characterised in that the operational amplifier (22) is connected via a resistor (21) to the input of the stage, and that in the direction of the output, the diode is followed by a capacitor (24), where, between the diode and the capacitor, a connection to earth is provided via a resistor (26), and between the operational amplifier and the capacitor a connection to the positive pole of the operating current source is provided via a resistor (28).

8. A device as claimed in Claim 4, characterised in that the highpass filter (13) has a cut-off frequency in the range between 2 kHz to 4 kHz, and that the low-pass filter (16) has a cut-off frequency in the range between 200 Hz to 1 kHz.

**Revendications**

1. Procédé pour la transmission d'informations acoustiques sous forme de vibrations tactiles qui sont obtenues dans un vibrateur (10) à partir des informations totales (signal de bas) amplifiées, transformée en une suite de signaux életriques, et à partir d'un signal dérivé de la fraction à haute fréquence contenue dans les informations totales, caractérisé en ce que la dérivation du signal à partir de la fraction à haute fréquence s'effectue par la formation des fréquences de différence des composantes du signal dont se compose la fraction à hautes fréquences.

2. Procédé selon la revendication 1, caractérisé en ce que la dérivation s'effectue au moyen d'un étage (15) à caractéristique non linéaire, notamment à caractéristique quadratique.

3. Procédé selon la revendication 1, caractérisé en ce que les fractions à haute fréquence sont prélevées de l'amplificateur, prévu pour la suite de signaux, entre un préamplificateur (2) et un amplificateur terminal (9), appliquées à un étage (15) formant des fréquences totales et des fréquences de différence et, enfin, portées à un niveau correspondant au niveau du signal de base et amenées ensemble avec ce signal à l'amplificateur terminal, muni d'un élément de réglage du volume (8), et que le signal mixte est transformé ensuite en vibrations tactiles dans un vibrateur (10).

4. Dispositif pour la mise en oeuvre du procédé selon la revendication 3, où les informations acoustiques sont recueillies dans un microphone (1) raccordé à un préamplificateur (2) qui est connecté à travers un amplificateur terminal (9) à un vibrateur (10) et où un canal de signal supplémentaire est branché en dérivation entre les deux parties d'amplificateur et contient, l'un après l'autre, un filtre passe-haut (13), un amplificateur (14), un étage (15) à caractéristique non linéaire, un filtre passe-bas (16) et un autre amplificateur (17).

5. Dispositif selon la revendication 4, caractérisé en ce que l'étage (15) à caractéristique non linéaire contient un amplificateur opérationnel (22) suivi d'une diode (23).

6. Dispositif selon la revendication 5, caractérisé en ce que l'amplificateur opérationnel présente une tension d'alimentation minimale de 3 volts et une fréquence limite supérieure de 20 kHz pour un gain de 30 dB et que la diode est une diode au silicium petit signal d'une puissance admissible de 400 mW, l'amplificateur opérationnel étant shunté par deux diodes montées tête-bêche en série avec une résistance.

7. Dispositif selon les revendications 5 et 6, caractérisé en ce que l'amplificateur opérationnel (22) est relié à travers une résistance (21) à l'entrée de l'étage et que la diode est suivi par un condensateur (24) en direction de la sortie, une liaison à la masse étant prévue, à travers une résistance (26), entre la diode et le condensateur et une connexion au pôle positif de la source de tension de service étant prévue, en outre, à travers une résistance (28), entre l'amplificateur opérationnel et le condensateur.

8. Dispositif selon la revendication 4, caractérisé en ce que le filtre passe-haut (13) possède une fréquence limite située dans le domaine de 2 kHz à 4 kHz et le filtre passe-bas (16) possède une fréquence limite située dans le domaine de 200 Hz à 1 kHz.

FIG 1

FIG 2